Europäisches Patentamt

European Patent Office

Office européen des brevets

(19)

(11) **EP 0 664 471 B1**

(12) **FASCICULE DE BREVET EUROPEEN**

(45) Date de publication et mention
de la délivrance du brevet:
**01.09.1999 Bulletin 1999/35**

(51) Int Cl.⁶: **G02B 27/00**, G02B 27/30,
A42B 3/04

(21) Numéro de dépôt: **95400105.3**

(22) Date de dépôt: **19.01.1995**

(54) **Dispositif de contrôle du réglage de position et d'orientation d'un casque par rapport à la tête d'un porteur du casque**

Vorrichtung zum Kontrollieren der Regelung von Position und Orientierung eines Helmes in bezug auf den Kopf seines Trägers

Device for checking the regulation of the position and orientation of a helmet in proportion to the head of the wearer

(84) Etats contractants désignés:
**DE GB**

(30) Priorité: **25.01.1994 FR 9400768**

(43) Date de publication de la demande:
**26.07.1995 Bulletin 1995/30**

(73) Titulaire: **SEXTANT AVIONIQUE
92360 Meudon-la-Forêt (FR)**

(72) Inventeurs:
• **Barbier, Bruno
F-92402 Courbevoie Cedex (FR)**
• **Lach, Patrick
F-92402 Courbevoie Cedex (FR)**
• **Leger, Alain
F-92402 Courbevoie Cedex (FR)**

(74) Mandataire: **Simonnet, Christine
Thomson-CSF Propriété Intellectuelle,
13, Avenue du Président Salvador Allende
94117 Arcueil Cédex (FR)**

(56) Documents cités:
**FR-A- 2 237 215        FR-A- 2 522 804**

**Description**

L'invention se situe dans le domaine des outillages destinés à adapter un système optronique monté sur un casque à la morphologie particulière du porteur du casque. Cette adaptation constitue la procédure de personnalisation du casque. De tels casques sont utilisés en particulier en aéronautique militaire.

Les équipements de tête en aéronautique militaire sont essentiellement constitués du casque, dont la fonction première est de protéger le pilote. Le casque sert également de support à des équipements respiratoires et auditifs, et à un système de visualisation, qui présente au pilote des informations par l'intermédiaire d'une source d'image (tube à rayons cathodiques, matrice à cristal liquide,...) et d'un système optique de collimation.

Le casque doit être conçu et adapté à la morphologie du pilote pour que l'utilisation des équipements de tête soit optimale. L'adaptation de chacun des casques à la morphologie de son porteur se fait lors de la procédure de personnalisation. Lors de cette procédure le casque doit être rigoureusement positionné et orienté par rapport à des repères anatomiques du sujet.

Dans l'état actuel de la technique, la procédure de personnalisation nécessite plusieurs opérations pour identifier précisément la morphologie du pilote. La fabrication du casque peut se faire à partir de ces informations morphologiques, mais ces opérations sont longues et imprécises à cause de la difficulté de mesurer avec précision les caractéristiques de la tête.

D'autres procédures demandent une participation active du sujet pour qu'il positionne sa tête, par rapport à des repères prédéfinis du casque ou d'un outillage associé. Lors de ces procédures le pilote risque de prendre des postures inconfortables, qui ne correspondent pas à des positions fonctionnelles qu'il adoptera lors de l'utilisation de l'équipement. Ces contraintes nuisent en particulier à l'efficacité ultérieure du système de visualisation qui se montera sur le casque.

FR-A-2 237 215 montre une lunette autocollimatrice conventionnelle comportant deux réticules de forme complémentaire. Le réglage s'effectue par superposition des images des deux réticules.

FR-A-2 522 804 montre un dispositif de mesure de la direction du regard de l'oeil dû à sa rotation dans son orbite, solidaire d'un casque et comportant des moyens pour imager la pupille de l'oeil sur une mire.

Le but de la procédure de personnalisation est de placer dans un même plan P deux diaphragmes optiques, un premier et un second. Le premier est constitué par la pupille de l'oeil du porteur du casque. Le second est la pupille du système optique qui va être monté sur le casque. Cette dernière se trouve dans un plan P qui n'a pas d'existence matérielle mais dont on connaît la position théorique par rapport à des repères pris sur le casque, par exemple les points de fixation du système optronique de visualisation.

Il est impératif que la pupille optique de ce système soit placée dans le même plan que la pupille de l'oeil du porteur, et que les deux diaphragmes constitués par les deux pupilles soient centrés l'un sur l'autre, quand le sujet regarde dans une direction de référence $\vec{u}$. Celle-ci est en général matérialisée par une cible collimatée et est située dans un plan horizontal, passant par la ligne des yeux du porteur lors de la procédure de personnalisation.

Le but de l'invention est de simplifier et raccourcir la procédure de personnalisation. Il est aussi de conserver au porteur une aisance et un confort au cours de cette procédure, qui soient au moins équivalents à l'aisance et au confort qu'il aura lorsqu'il sera équipé. Il est enfin d'améliorer la précision du positionnement du casque.

L'objet de l'invention est relatif à un outillage, comportant des moyens optiques et un capteur d'images, qui garde une position fixe par rapport à un casque de personnalisation ou par rapport au casque réel. Un moyen simple de réaliser cette condition est d'utiliser, pour tenir l'outillage, une structure mécanique rigide qui se fixe sur le casque, par exemple en utilisant les points de fixation prévus du système optronique. C'est ce moyen simple qui est utilisé dans le mode préféré de réalisation. L'outillage peut par ailleurs être soutenu par un bras extérieur de façon à augmenter le confort du porteur. L'outillage pourrait ne pas avoir de lien mécanique direct par rapport au casque. L'essentiel est que l'outillage suive en permanence les mouvements du casque en ayant par rapport à lui une position fixe connue. C'est cet outillage qui permet de repérer et contrôler facilement la position du casque réel ou d'un casque de personnalisation, par rapport à la position de la pupille du porteur. Il permet aussi de repérer facilement l'orientation de l'axe visuel du porteur.

Lorsque la position et l'orientation du casque réel ou du casque outillage ont été réglées de telle sorte que la pupille de l'oeil du porteur coïncide avec le plan théorique P contenant la pupille du dispositif optronique à monter sur le casque, on peut compléter le casque, par exemple par injection d'une mousse durcissable qui s'adaptera à la morphologie de la tête du porteur. Le moulage en position ainsi réalisé restera attaché au casque s'il s'agit du casque réel ou sera utilisé pour conformer un ou plusieurs autres casques si l'on a utilisé un casque spécial de personnalisation. Dans ce dernier cas, il sera également possible d'utiliser le premier moulage réalisé sur la tête du porteur pour réaliser d'autres moulages adaptés à la morphologie du porteur, ces moulages nécessitant pour leur mise en oeuvre des conditions que ne supporterait pas le porteur (température, pression, produits nocifs...).

En résumé, l'invention est relative à un outillage pour régler et vérifier que la position d'une pupille oculaire d'un porteur d'un casque ayant dans une position de repos une position de regard définie par une direction axiale $\vec{u}$, le casque étant destiné à recevoir à un emplacement matérialisé par des repères du casque des moyens optroniques ayant eux-mêmes une pupille optique dont la position par rapport aux repères est connue, ces moyens optroniques ayant également un axe optique $\vec{v}$ dont la direction par rapport aux repères est connue, coïncide avec la position de la pupille des moyens optroniques et que la direction axiale $\vec{u}$ du regard du porteur fait par rapport à l'axe optique $\vec{v}$ des moyens optroniques un angle prédéterminé, outillage caractérisé en ce qu'il comporte :

- des premiers moyens optiques destinés à former une image de la pupille de l'oeil sur un premier écran $E_1$ comportant une première mire, l'ensemble mécanique formé par les premiers moyens optiques et le premier écran occupant une position fixe connue par rapport au casque, cette position étant telle qu'une image nette de la pupille de l'oeil du porteur centrée sur la première mire ne peut être obtenue que si la position de la pupille de l'oeil coïncide avec la position de la pupille des moyens optroniques à monter sur le casque,
- des seconds moyens optiques destinés à faire correspondre de façon biunivoque à chaque direction spatiale un point sur un second écran $E_2$ comportant une seconde mire représentant une figure, les seconds moyens optiques et le second écran occupant une position fixe connue par rapport au casque, cette position étant telle qu'une figure collimatée située par rapport au porteur du casque dans la direction $\vec{u}$ ne puisse avoir une image superposée à la seconde mire que si ces seconds moyens optiques sont orientés dans une direction dans laquelle les directions axiales $\vec{u}$ du regard du porteur et $\vec{v}$ de l'axe optique des moyens optroniques sont orientées l'une par rapport à l'autre dans la direction prédéterminée.

De façon avantageuse dans le mode de réalisation préféré les écrans $E_1$ et $E_2$ sont confondus en un seul. Les premier et second moyens optiques comportent donc des moyens optiques communs.

Dans le mode de réalisation préféré le casque utilisé est un casque spécial de personnalisation monté sur un ensemble mécanique permettant d'une part une translation du casque dans trois directions formant un trièdre orthonormé et d'autre part une rotation du casque sur lui-même autour de trois axes formant un trièdre orthonormé.

De tels ensembles mécaniques sont bien connus dans l'art. Cet aspect ne sera pas abordé dans la description qui sera faite ci-après d'un mode de réalisation de l'invention. Sa description sera faite en regard des dessins annexés. Cette description fera apparaître d'autres détails et avantages de l'invention. Dans les dessins :

- la figure 1 est un schéma destiné à illustrer le mode préféré de réalisation de l'invention ;
- la figure 2 représente la mire d'un écran, ainsi qu'un oeil fictif bien positionné par rapport à la mire ;
- la figure 3 représente les trajets optiques, à partir du porteur du casque et d'une cible auxiliaire à travers les premier et second moyens optiques.

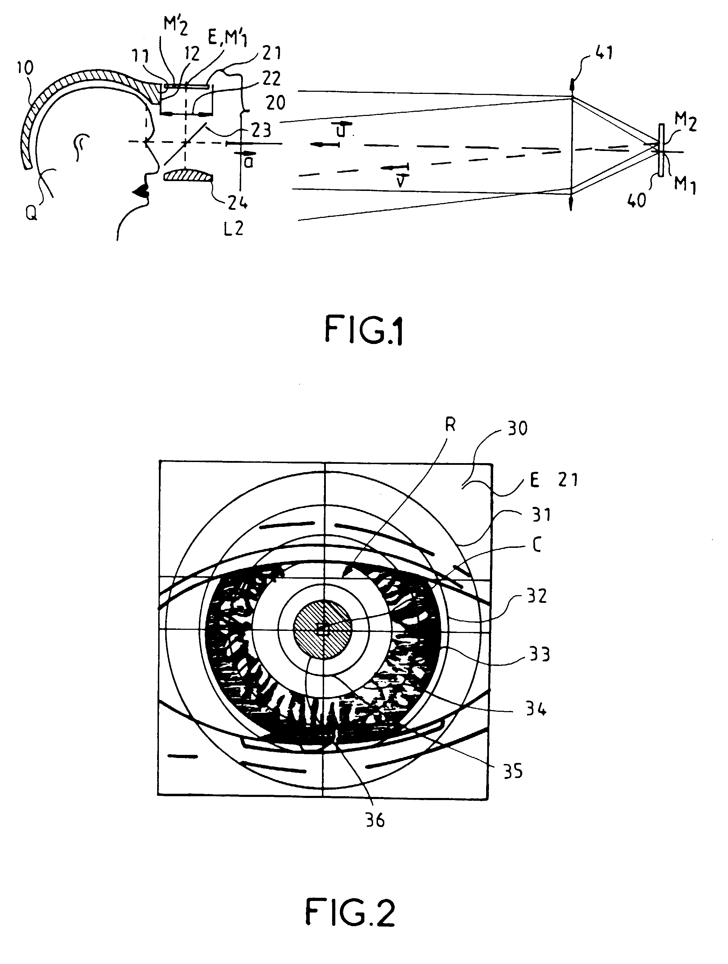

La figure 1 représente un schéma destiné à illustrer le mode préféré de réalisation de l'invention.

Dans ce mode de réalisation, l'outillage 20 selon l'invention est fixé à une structure de casque 10, qui reproduit la forme d'un casque réel et est destinée après vérification de sa position et de son orientation correcte par rapport à la tête du porteur représentée en Q, à fournir une forme pour la partie extérieure d'un moulage dont la forme intérieure sera constituée par la forme extérieure de la tête du porteur Q. Ce moulage sera ensuite après reproduction, éventuellement dans une matière différente, reporté dans la structure d'un ou plusieurs casques qui seront, par ce report, adaptés à la morphologie du porteur. Comme expliqué plus haut, cette structure 10 est fixée de façon connue à un ensemble mécanique non représenté. Cet ensemble mécanique permet une translation de la structure 10 selon trois axes avec un débattement suffisant pour permettre les réglages de position, en hauteur, latéraux et avant arrière. Des moyens mécaniques liés à la structure et ayant par rapport à cette structure la même position que les moyens de fixation des moyens optroniques devant se monter sur un casque, sont représentés sur la figure 1 d'une part par une face de référence 11 et d'autre part par un ensemble de pions d'ancrage 12 dont un seul est représenté. Cette face de référence 11 et ces pions d'ancrage 12 sont utilisés pour fixer les premier et second moyens optiques ainsi que l'écran unique E à l'aide d'une structure porteuse rigide non représentée.

Les moyens de fixation 11-12 déterminent la position, par rapport à la structure 10, d'un plan P contenant la pupille optique des moyens optroniques et leur direction optique axiale représentée sur la figure 1 par un vecteur $\vec{v}$. Cette position est connue au départ par les calculs de conception, et elle peut être contrôlée par des moyens de mesure en eux-mêmes connus.

Comme déjà expliqué plus haut, on rappelle que l'outillage 20 permet de contrôler en temps réel, d'une part le centrage de la pupille d'oeil sur la position théorique de la pupille instrumentale du système de visualisation, et d'autre part l'orientation de la direction centrale de ce système par rapport à une direction de référence. Au cours de cette procédure le système de visualisation n'est pas monté sur le casque ou la structure 10, mais après la procédure on aura la certitude qu'il viendra se positionner correctement par rapport aux repères anatomiques du sujet, lorsqu'on le fixera sur le casque.

Pour ne pas brouiller de façon inutile la compréhension, des pièces mécaniques utilisées, d'une part pour relier entre eux de façon fixe les éléments entrant dans la réalisation de l'outillage et d'autre part l'ensemble de ces éléments aux moyens mécaniques 11, 12 de la structure 10, n'ont pas été représentées. Lorsqu'on dit que la position relative des divers éléments constituant l'outillage est fixe entre eux et par rapport à la structure 10, cela n'exclut pas que cette position soit néanmoins réglable. Le réglage permet d'utiliser le même outillage pour différents types de casques. Pour un même type de casque la même position de réglage peut être utilisée.

L'outillage 20 comprend les premier et second moyens optiques ainsi que les deux écrans $E_1$ et $E_2$. Ces deux écrans sont dans le mode de réalisation ici décrit confondus en un seul E référencé 21. Dans le mode de réalisation préféré, l'écran est constitué par une matrice de capteur CCD (charge coupled device). Cet écran permet de récupérer et d'observer l'image formée sur cet écran, sur un moniteur de contrôle d'accès facile pour un opérateur de personnalisation. Naturellement cet écran pourrait être réalisé par tout moyen connu par exemple par une surface diffusante dont on peut observer les images soit directement soit par l'intermédiaire d'un oculaire.

Des points particuliers peuvent être prématérialisés sur la surface de cet écran. Dans le cas du mode de réalisation préféré, ces points particuliers sont matérialisés par une mire électronique qui se superpose à l'image fournie par les capteurs électrooptiques. C'est cette mire électronique qui permettra de vérifier par comparaison l'emplacement correct d'images formées sur l'écran et partant l'emplacement correct de la structure 10 par rapport à la tête du porteur Q. Les premiers moyens optiques sont destinés à former une image de la pupille de l'oeil du porteur Q sur l'écran E 21. Dans le cas de la réalisation ils comportent un miroir plan 23 constitué dans ce cas par une lame partiellement réfléchissante, et par un moyen de focalisation constitué dans le cas de la réalisation par une lentille $L_1$ 22. Lorsque l'oeil du porteur Q est correctement positionné dans le plan P de la pupille des moyens optroniques, le centre de la pupille de l'oeil doit se trouver en un point C préalablement repéré sur l'écran.

Dans le cas de la réalisation la mire de position comprend outre le point C des cercles concentriques centrés sur C. Ces cercles permettent de s'assurer que la pupille est bien centrée sur C. Un exemple de l'image à obtenir est représenté figure 2. Sur cette figure, les différents cercles concentriques sont repérés 31 à 36. Une image d'un oeil fictif bien centré est superposée à la mire 30 constituée des cercles 31 à 36 et du point C. Ce premier réglage est obtenu par translation 3 axes de la structure 10.

Les seconds moyens optiques sont également représentés figure 1. Ils comportent la lentille $L_1$ 22 ainsi qu'un miroir qui dans ce cas est constitué par la lame partiellement réfléchissante 23. Les moyens 22 et 23 sont donc communs aux premier et second moyens optiques. Les seconds moyens optiques comportent en outre un miroir sphérique 24. Ce sont ces seconds moyens optiques qui vont permettre de s'assurer que l'orientation relative de l'axe du regard du porteur et de l'axe des moyens optroniques est correcte.

A cette fin, on utilise un moyen auxiliaire constitué par une cible collimatée. Cette cible dans le cas de la réalisation est constituée par un écran 40 comportant deux points matérialisés $M_1$ et $M_2$. L'écran est mobile verticalement. Un ensemble de collimation 41 mobile avec l'écran permet pour un observateur de transformer de façon biunivoque chaque point de l'écran en une direction dans l'espace.

Au cours de la procédure de personnalisation il est demandé au porteur Q de regarder le point $M_1$. De la sorte, la direction de son regard $\vec{u}$ est matérialisée sur l'écran 40 par le point $M_1$. Le point $M_2$ correspond à la direction $\vec{v}$.

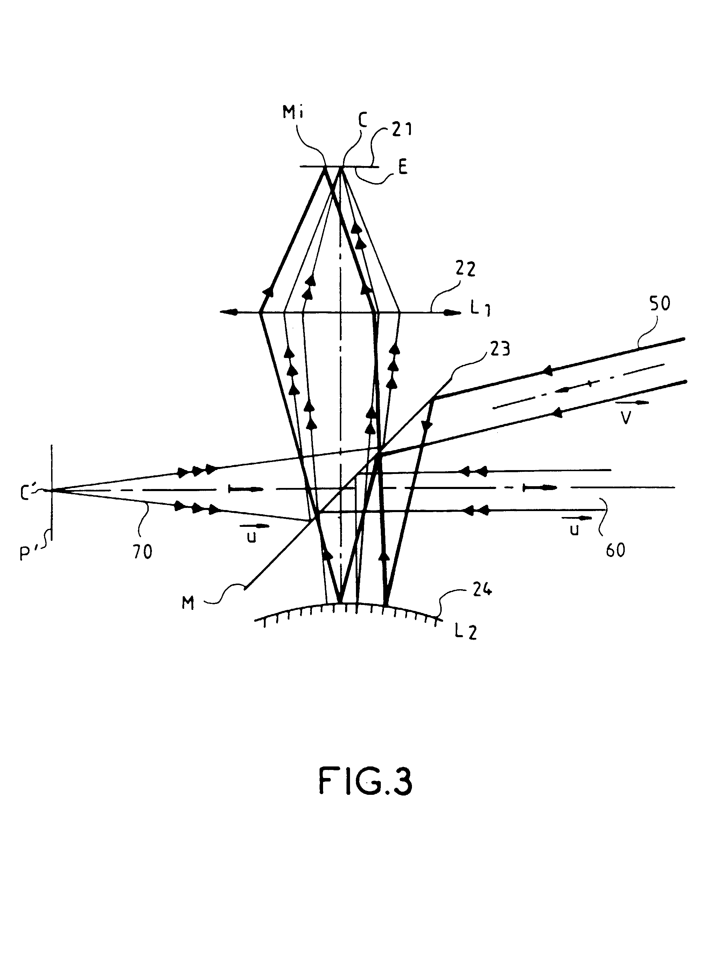

Les seconds moyens optiques sont agencés pour faire correspondre de façon biunivoque aux directions $\vec{u}$ et $\vec{v}$ des points $M'_1$ et $M'_2$ sur l'écran unique E 21. Ces points sont prépositionnés et font partie de la mire 30. Dans le mode préféré de réalisation, la direction $\vec{u}$ est transformée par les seconds moyens optiques en une direction qui correspond à l'axe optique des seconds moyens optiques. La direction $\vec{v}$ est transformée par les seconds moyens optiques en une direction $\vec{r}$. Cette direction $\vec{r}$ correspond de façon biunivoque sur l'écran $E$ au point $M'_2$. La direction $\vec{r}$ dans l'une des mires 30 utilisée pour personnaliser un type de casque a une direction qui correspond à une direction $\vec{v}$ faisant avec la direction $\vec{u}$ un angle de 3° dans un plan vertical comprenant la direction $\vec{u}$. Le fonctionnement optique de l'outillage selon l'invention sera maintenant expliqué en référence à la figure 3.

Cette figure représente la combinaison des trois trajets optiques qui se combinent dans l'outillage réalisé.

Le premier 50 dont les rayons sont repérés par un signe flèche est constitué par les rayons en provenance de la direction $\vec{v}$ correspondant au point $M_2$ sur l'écran 40. Ils se réfléchissent sur le miroir constitué par la lame semi-réfléchissante 23 en direction du miroir sphérique $L_2$ 24. Après réflexion sur $L_2$, les rayons traversent la lame 23 et sont focalisés par la lentille $L_1$ 22 vers le point $M'_2$ de l'écran E 21.

Le second 60 dont les rayons sont repérés par deux signes flèches situés l'un derrière l'autre, est constitué par les rayons en provenance de la direction $\vec{u}$ correspondant au point $M_1$ sur l'écran 40. Après réflexion sur le miroir 23, ils se réfléchissent sur le miroir sphérique $L_2$ 24 traversent la lame 23 et sont focalisés par la lentille 22 sur l'écran E au point C.

Le troisième 70 provient de l'oeil du porteur. Ses rayons sont matérialisés par trois flèches qui se suivent. Après réflexion sur le miroir 23 les rayons sont focalisés au point C de l'écran E 21 par la lentille $L_1$ 22.

Tous les points de focalisation indiqués ci-dessus pour l'écran E 21 sont les points de focalisation obtenus lorsque le casque et donc l'outillage optique qui lui est lié, sont correctement positionnés en translation et rotation.

La procédure de réglage se déroule alors comme suit :

- le porteur est placé dans une position de fonction, par exemple sur un siège dont la géométrie est représentative de l'application recherchée,
- l'unique tâche du sujet consiste à fixer tout au long de la procédure le point particulier $M_1$ de la cible qui est localisée devant lui, dans la direction $\vec{u}$ connue dans le référentiel du laboratoire,
- on s'assurera que dans ces conditions le plan de référence anatomique (plan de FRANKFORT) est sensiblement situé à l'horizontal. S'il présente une trop grande divergence, il faut opérer une correction de la posture de la tête,
- le porteur immobilise la tête par rapport au référentiel du laboratoire, dans cette position de fonction,
- un opérateur règle la position et l'orientation du casque 10 équipé de l'outillage. Le casque 10 est également muni d'une garniture ébauche maintenue souple à une température convenable. L'ensemble peut être monté sur une machine tridimensionnelle 3 translations/3 rotations manipulable par l'opérateur.

Le réglage consiste essentiellement à :

- translater et orienter le casque pour obtenir une image nette et centrée de la pupille d'oeil du sujet sur l'écran E (figure 2). L'ouverture de l'objectif $L_1$ est choisie pour que sa profondeur de champ soit de l'ordre de grandeur de la précision de positionnement souhaitée de la pupille optique du système de visualisation par rapport à la pupille d'oeil. Le centrage se fait par rapport aux repères qui se superposent à l'écran E, c'est-à-dire les cercles concentriques 31 à 36 et le point C,
- translater et orienter le casque de telle sorte que le point image de $M_2$ visualisé sur l'écran E 21 se superpose avec le point $M'_2$ de la mire.

Un mouvement de translation ou de rotation peut agir simultanément sur la netteté de l'image de la pupille sur l'écran E 21 son centrage et la position relative de l'image des points $M_2$ et $M_1$ sur l'écran E. L'opérateur doit agir alternativement sur les mouvements de rotation et de translation pour converger simultanément vers la bonne position et la bonne orientation du casque.

Une fois que les trois critères de réglage sont satisfaits, le casque est immobilisé et le matériau de garniture est injecté.

Lors de cette opération, l'outillage permet de contrôler l'évolution de la qualité de la personnalisation, en observant en temps réel, les dérives éventuelles du centrage de la pupille d'oeil sur C, de sa netteté, et de la superposition des points $M'_2$ et C, quand le sujet fixe le point $M_1$.

Un contrôle additionnel est de vérifier que sur l'écran E 21 le point image du point $M_1$ de la cible, se superpose bien avec le repère C.

## Revendications

1. Outillage (20) pour régler et vérifier que la position d'une pupille oculaire d'un porteur d'un casque ayant dans une position de repos une direction de regard définie par une direction axiale $\vec{u}$, le casque étant destiné à recevoir à un emplacement matérialisé par des repères du casque des moyens optroniques ayant eux-mêmes une pupille optique dont la position par rapport aux repères est connue, ces moyens optroniques ayant également un axe optique $\vec{v}$ dont la direction par rapport aux repères est connue, coïncide avec la position de la pupille des moyens optroniques et que la direction axiale $\vec{u}$ du regard du porteur fait par rapport à l'axe optique $\vec{v}$ des moyens optroniques un angle prédéterminé, outillage caractérisé en ce qu'il comporte :

   - des premiers moyens optiques (22, 23) destinés à former une image de la pupille de l'oeil sur un premier écran $E_1$ (21) comportant une première mire (30), les premiers moyens optiques et le premier écran occupant une position fixe connue par rapport au casque, cette position étant telle qu'une image nette de la pupille de l'oeil du porteur centrée sur la première mire ne peut être obtenue que si la position de la pupille de l'oeil coïncide avec la position de la pupille des moyens optroniques à monter sur le casque,
   - des seconds moyens optiques (22, 23, 24) destinés à faire correspondre de façon biunivoque à chaque direction spatiale, un point sur un second écran $E_2$ (21) comportant une seconde mire (30) représentant une figure, les seconds moyens optiques et le second écran occupant une position fixe connue par rapport au casque, cette position étant telle qu'une figure collimatée située par rapport au porteur du casque dans la direction $\vec{u}$ ne puisse avoir une image superposée à la seconde mire que si ces seconds moyens optiques sont orientés dans une direction dans laquelle les directions axiales $\vec{u}$ du regard du porteur et $\vec{v}$ de l'axe optique des moyens optroniques sont orientées l'une par rapport à l'autre dans la direction prédéterminée.

2. Outillage selon la revendication 1, caractérisé en ce que les premiers moyens optiques comprennent un miroir (23) et un moyen de focalisation (22).

3. Outillage selon la revendication 1, caractérisé en ce que les seconds moyens optiques comprennent un miroir (23) et un moyen de focalisation (24, 22).

4. Outillage selon la revendication 1, caractérisé en ce que les écrans $E_1$ et $E_2$ sont confondus en un seul E (21) les premier et second moyens optiques comportant des moyens communs (23, 22).

5. Outillage selon la revendication 4, caractérisé en ce que les moyens communs comportent une lame partiellement transparente (23) et un moyen de focalisation (22) et en ce que l'un des deux moyens optiques comporte en outre un moyen complémentaire de focalisation (24).

6. Outillage selon la revendication 5, caractérisé en ce que le moyen complémentaire de focalisation est un miroir sphérique (24).

**Patentansprüche**

1. Einrichtung (20) zur Justierung und Überprüfung, ob die Lage einer Augenpupille eines Helmträgers, der in einer Ruhestellung eine durch eine axiale Richtung $\vec{u}$ definierte Blickrichtung einnimmt, wobei der Helm an einer durch Bezugspunkte des Helms materialisierten Stelle Optronikmittel aufnehmen soll, die ihrerseits eine optische Pupille besitzen, deren Lage bezüglich der Bezugsmarken bekannt ist, wobei diese optronischen Mittel auch eine optische Achse $\vec{v}$ besitzen, deren Richtung bezüglich der Bezugsmarken bekannt ist, mit der Lage der Pupille der Optronikmittel zusammenfallen, und ob die axiale Richtung $\vec{u}$ der Blickrichtung des Trägers bezüglich der optischen Achse $\vec{v}$ der Optronikmittel einen vorbestimmten Winkel einnimmt, dadurch gekennzeichnet, daß die Einrichtung aufweist:

- erste optische Mittel (22, 23), die ein Bild der Pupille des Auges auf einem ersten Bildschirm $E_1$ (21) erzeugen sollen, der ein erstes Fadenkreuz besitzt, wobei die Einheit aus den ersten optischen Mitteln und dem ersten Bildschirm eine bezüglich des Helms bekannte feste Stellung einnimmt, die so gewählt ist, daß ein scharfes Bild der Pupille des Auges des Trägers, das auf das erste Fadenkreuz zentriert ist, erst erhalten werden kann, wenn die Lage der Pupille des Auges mit der Lage der Pupille der Optronikmittel zusammenfällt, die auf dem Helm montiert werden sollen,
- zweite optische Mittel (22, 23, 24), die in eineindeutiger Weise jeder räumlichen Richtung einen Punkt auf einem zweiten Schirm $E_2$ (21) zuordnen sollen, der ein zweites, eine Figur darstellendes Fadenkreuz (30) enthält, wobei die zweiten optischen Mittel und der zweite Bildschirm eine bekannte feste Lage bezüglich des Helms einnehmen, die so gewählt ist, daß die kollimatierte Figur, die bezüglich des Trägers des Helms in der Richtung $\vec{u}$ liegt, erst dann ein mit dem zweiten Fadenkreuz überlagertes Bild ergibt, wenn diese zweiten optischen Mittel in einer Richtung ausgerichtet sind, in der die axiale Blickrichtung $\vec{u}$ des Trägers und die optische Achse $\vec{v}$ der Optronikmittel zueinander in der vorbestimmten Richtung ausgerichtet sind.

2. Einrichtung nach Anspruch 1, dadurch gekennzeichnet, daß die ersten optischen Mittel einen Spiegel (23) und ein Fokussiermittel (22) aufweisen.

3. Einrichtung nach Anspruch 1, dadurch gekennzeichnet, daß die zweiten optischen Mittel einen Spiegel (23) und ein Fokussiermittel (24, 22) aufweisen.

4. Einrichtung nach Anspruch 1, dadurch gekennzeichnet, daß die Schirme ($E_1$, $E_2$) von einem gemeinsamen Schirm E (21) gebildet werden, wobei die ersten und zweiten optischen Mittel gemeinsame Mittel (23, 22) enthalten.

5. Einrichtung nach Anspruch 4, dadurch gekennzeichnet, daß die gemeinsamen Mittel ein teilweise transparentes Plättchen (23) und ein Fokussiermittel (22) enthalten und daß eines der beiden optischen Mittel außerdem ein komplementäres Fokussiermittel (24) enthält.

6. Einrichtung nach Anspruch 5, dadurch gekennzeichnet, daß das komplementäre Fokussiermittel ein sphärischer Spiegel (24) ist.

**Claims**

1. Instrument (20) for checking and verifying that the position of an ocular pupil of a person wearing a helmet having, in a position of rest, a line of sight defined by an axial direction $\vec{u}$, the helmet being designed to receive optronic means at a location physically represented by reference points on the helmet, these optronic means themselves having an optical pupil, the position of which with respect to the reference marks is known, and also having an optical axis $\vec{v}$ whose direction with respect to the reference marks is known, coincides with the position of the pupil of the optronic means and that the axial direction $\vec{u}$ of the wearer's line of sight makes a predetermined angle with the optical axis $\vec{v}$ of the optronic means, which instrument is characterized in that it comprises:

-   first optical means (22, 23) designed to form an image of the pupil of the eye on a first screen E1 (21) having a first test pattern (30), the first optical means and the first screen occupying a known fixed position with respect to the helmet, this position being such that a sharp image of the pupil of the wearer's eye centred on the first test pattern can be obtained only if the position of the pupil of the eye coincides with the position of the pupil of the optronic means to be mounted on the helmet,

-   second optical means (22, 23, 24) designed to make a point on a second screen $E_2$ (21), having a second test pattern (30) representing a figure, in one-two-one correspondence with each spatial direction, the second optical means and the second screen occupying a known fixed position with respect to the helmet, this position being such that a collimated figure located, with respect to the person wearing the helmet, in the direction $\vec{u}$ can have an image superimposed on the second test pattern only if these second optical means are oriented in a direction in which the axial directions $\vec{u}$ of the wearer's line of sight and $\vec{v}$ of the optical axis of the optronic means are oriented, one with respect to the other, in the predetermined direction.

2.  Instrument according to Claim 1, characterized in that the first optical means comprise a mirror (23) and a focusing means (22).

3.  Instrument according to Claim 1, characterized in that the second optical means comprise a mirror (23) and a focusing means (24, 22).

4.  Instrument according to Claim 1, characterized in that the screens $E_1$ and $E_2$ merge into a single screen E (21), the first and second optical means comprising common means (23, 22).

5.  Instrument according to Claim 4, characterized in that the common means comprise a partially transparent plate (23) and a focusing means (22) and in that one of the two optical means furthermore comprises a complementary focusing means (24).

6.  Instrument according to Claim 5, characterized in that the complementary focusing means is a spherical mirror (24).

FIG.1

FIG.2

FIG.3